# EUROPÄISCHE PATENTSCHRIFT

(11) **EP 1 861 031 B1**
(45) Veröffentlichungstag und Bekanntmachung des Hinweises auf die Patenterteilung: **14.04.2010**
(21) Anmeldenummer: 06705403.1
(22) Anmeldetag: 20.03.2006
(51) Int. Cl.: A61B 17/80

(54) **KNOCHENPLATTE**
BONE PLATE
PLAQUE D'OSTEOSYNTHESE

(30) Priorität: 24.03.2005 CH 517052005
(43) Veröffentlichungstag der Anmeldung: 05.12.2007
(73) Patentinhaber: Medartis AG, 4057 Basel (CH)
(72) Erfinder: PFEFFERLE, Joachim, 79244 Münstertal (DE); THIEL, Dirk, 79219 Staufen (DE); CORNELIUS, Carl-Peter, 89075 Ulm (DE)
(74) Vertreter: Müller, Christoph Emanuel
(86) Internationale Anmeldenummer: PCT/CH2006/000163
(87) Internationale Veröffentlichungsnummer: WO 2006/099766

(56) Entgegenhaltungen:
- WO-A-00/66012
- WO-A-01/19268
- WO-A-01/30251
- WO-A-95/16403
- WO-A-2004/086990
- FR-A- 742 618
- US-A- 5 690 631
- US-A- 6 077 266

## Beschreibung

Die Erfindung betrifft eine Knochenplatte gemäss dem Oberbegriff des unabhängigen Patentanspruchs.

Knochenplatten sind in unzähligen verschiedenen Ausführungen heute im Einsatz und bieten eine nicht mehr verzichtbare Hilfe bei der Osteosynthese, insbesondere bei Frakturen, bei denen die beiden Knochenfragmente diesseits und jenseits einer Fraktur wieder zusammenwachsen müssen. Dabei dient die Knochenplatte dazu, dass die Knochenfragmente in einer gewünschten Relativposition zueinander gehalten werden, sodass der Knochen wie gewünscht zusammenwachsen kann.

Ein spezielles Beispiel für eine solche Knochenplatte ist aus der WO-A-01/19268 bekannt. Bei einzelnen Ausführungsbeispielen der dort beschriebenen Knochenplatte (siehe Fig. 2 und Fig. 3 in WO-A-01/19268) verläuft die Lochachse von einzelnen Plattenlöchern schräg relativ zur Plattenunterseite, während die Plattenoberseite und die Plattenunterseite parallel zueinander verlaufen. Die Innenwand des Plattenlochs weist einen umlaufenden Grat auf, welcher beim Eindrehen der Knochenschraube umgeformt wird, sodass die Schraube im Plattenloch "verblockt" ist, also in der Eindrehstellung gesichert ist.

Die in der WO-A-01/19268 gezeigten Knochenplatten sind speziell für Röhrenknochen, z.B. die Tibia, offenbart. Solche Knochenplatten weisen eine erhebliche Materialstärke auf, sodass es aufgrund dieser Materialstärke normalerweise kein Problem darstellt, den Schraubenkopf im Schraubenloch zu versenken, auch wenn die Schraube so eingedreht wird, dass die tatsächliche Eindrehrichtung von der "Solleindrehrichtung", also von der Richtung der Lochachse, abweicht, was innerhalb gewisser Grenzen möglich ist. Obendrein ist das Operationsfeld bei Röhrenknochen wie z.B. der Tibia für den Chirurgen zumeist gut zugänglich.

Schwieriger gestaltet sich das Versenken des Schraubenkopfes bei "dünnen" Knochenplatten mit einer wesentlich geringeren Plattenstärke, nämlich insbesondere dann, wenn die tatsächliche Eindrehrichtung der Knochenschraube von der "Solleindrehrichtung" abweicht, weil dann aufgrund der geringeren Plattenstärke die axiale Länge des Lochs nicht sehr gross ist. Solche vergleichsweise "dünne" Knochenplatten sind aufgrund des geringen Weichgewebeangebots und auch aus ästhetischen Gründen insbesondere im maxillo-facialen Bereich aber die Regel.

Aus der WO-A-95/16403 ist eine Knochenplatte bekannt, bei der die Lochachsen der Plattenlöcher unter einem von 90° verschiedenen Winkel relativ zur Ebene der Plattenunterseite verlaufen. Um die Plattenlöcher herum ist auf der Plattenoberseite jeweils eine Erhöhungen vorgesehen. Zur Befestigung der Knochenplatte wird zunächst mit einem geeigneten Bohrwerkzeug, welches in dem schräg verlaufenden Plattenloch geführt wird, eine Vorbohrung im Knochen erstellt, die genau in Richtung der Lochachse verläuft. Beim anschliessenden Eindrehen der Knochenschraube wird die Knochenschraube in der Vorbohrung geführt und das Eindrehen erfolgt in der Solleinschraubrichtung, nämlich in Richtung der Lochachse. Dadurch kann der zylindrische Schraubenkopf vollständig in der Erhöhung der Knochenplatte aufgenommen werden. Eine Art Verblockung der Knochenschraube in vollständig eingeschraubter Position ist hier derart vorgeschlagen, dass in der Aussenwand des zylindrischen Schraubenkopfs Vertiefungen vorgesehen sind und nach vollständig erfolgtem Einschrauben mit Hilfe eines geeigneten Werkzeugs die Überhöhung der Knochenplatte in diese Vertiefung hinein deformiert wird. Die Knochenschraube kann aber bei der Knochenplatte aus der WO-A-95/16403 nur genau in Richtung der Lochachse eingeschraubt werden, da ansonsten der zylindrische Schraubenkopf nicht in der Überhöhung aufgenommen werden kann. Deshalb muss auch eine Vorbohrung im Knochen erstellte werden, die genau die gewünschten Einschraubrichtung vorgibt. Dies ist aber für den Chirurgen aufwändig (Erstellen der Vorbohrung, etc.) und erlaubt auch praktisch keine Abweichung der Eindrehrichtung von der Solleindrehrichtung.

Häufig stellt sich bei schräg verlaufenden Lochachsen und somit schräg verlaufender "Solleindrehrichtung" für den Chirurgen aber das Problem, dass er - insbesondere bei einem beengten Operationsfeld, wie dies im maxillo-facialen Bereich häufig der Fall ist - die "Solleindrehrichtung" nicht gut erkennen kann. Wenn die Abweichung der tatsächlichen Eindrehrichtung der Knochenschraube von der Solleindrehrichtung in gewissem Umfang abweicht, würde sich der Schraubenkopf bei der zuvor erläuterten Knochenplatte nicht im Plattenloch versenken lassen. Ein Vorbohren der Solleindrehrichtung ist aber aufwändig und von der Zugänglichkeit gerade im maxillo-facialen Bereich problematisch. Ohne Vorbohren ergibt sich jedoch für den Chirurgen die Schwierigkeit, die "Solleindrehrichtung gut zu erkennen, insbesondere dann, wenn ein interoraler Eingriff (Zugang nur durch den Mund) vorgenommen werden soll, aber sie kann sich auch bei einem Zugang mit Hautschnitt (Inzision) ergeben. Der Ort eines Hautschnitts kann nämlich aufgrund des Verlaufs der Nerven im maxillo-facialen Bereich nicht einfach beliebig so gewählt werden, dass der Schraubendreher dann senkrecht durch die Inzision geführt werden kann und auf die Knochenplatte stösst. Dies wäre zwar vom Handling her eine einfache Variante, lässt sich aber aufgrund des Verlaufs der Nerven häufig nicht realisieren. Die gleiche Schwierigkeit bei der Wahl des Ortes ergibt sich auch bei einem sogenannten "transbuccalen" Zugang, bei welchem quasi ein kleines Loch durch die Haut erzeugt wird (aber kein grösserer Schnitt), durch welches dann beispielsweise der Schraubendreher hindurch geführt wird. Die Platte selbst hingegen kann interoral eingebracht und am gewünschten Ort gehalten werden.

Die vorliegende Erfindung schlägt nun eine Knochenplatte vor, wie sie durch die Merkmale des unabhängigen Patentanspruchs charakterisiert ist. Vorteilhafte Ausführungsbeispiele der erfindungsgemässen Knochenplatte sind Gegenstand der abhängigen Patentansprüche.

Speziell verläuft die Plattenoberseite der erfindungsgemässen Knochenplatte zumindest im Bereich um das Plattenloch herum wenigstens teilweise nicht parallel zu der Plattenunterseite, sondern insbesondere senkrecht zur Lochachse. Diese Fläche erlaubt es dem Chirurgen, die Solleindrehrichtung der Knochenschraube besser zu erkennen und somit die Schraube wenigstens annähernd in der Solleindrehrichtung einzuschrauben, wodurch es in den meisten Fällen möglich ist, den Schraubenkopf vollständig oder beinahe vollständig im Plattenloch zu versenken. Das Plattenloch ist dabei so ausgebildet, dass eine in das Plattenloch eingeschraubte Knochenschraube unter einem Winkel aus einem vordefinierten Winkelbereich um den Elevationswinkel der Lochachse des Plattenlochs herum in dem Plattenloch verblockbar ist, und zwar nur durch Zusammenwirken des speziell ausgebildeten Plattenlochs mit der Knochenschraube, also ohne zusätzliche weitere Hilfsmittel. Das heisst, dass der Chirurg beim Eindrehen der Knochenschraube nicht vorbohren muss, was von der Zugänglichkeit insbesondere im maxillo-facialen Bereich häufig auch schwierig ist. Ausserdem erlaubt die Knochenplatte beim Einschrauben der Knochenschraube eine gewisse Schwankungsbreite um den Solleindrehwinkel herum, der insofern vorteilhaft ist, als es einerseits schwierig ist, eine Schraube ohne Vorbohren genau in Solleindrehrichtung einzudrehen, und anderseits weil manchmal auch das Knochenangebot bei einem Patienten so ist, dass die Schraube sich besser unter einem von der Solleindrehrichtung abweichenden Winkel im Knochen fixieren lässt. Mit der erfindungsgemässen Knochenplatte wird insbesondere ein interoraler Zugang begünstigt, wodurch in vielen Fällen ein Hautschnitt unterbleiben kann. Ist die Schraube eingedreht, bedarf es auch keiner besonderen Massnahmen mehr zur Verblockung, weil das jeweilige Plattenloch eben so ausgebildet ist, dass die Verblockung rein durch das Zusammenwirken des speziell ausgebildeten Plattenlochs mit der Knochenschraube zustande kommt, also ohne weitere Hilfsmittel (wie z.B. Spreizschrauben, die den Schraubenkopf aufspreizen, oder separate Deckel, die dann eingeschraubt werden müssen, um den Schraubenkopf niederzuhalten).

Bei einem vorteilhaften Ausführungsbeispiel der erfindungsgemässen Knochenplatte kann der vordefinierte Winkelbereich um den Elevationswinkel der Lochachse herum etwa 15° betragen.

Ein Ausführungsbeispiel der erfindungsgemässen Knochenplatte umfasst mehrere Plattenlöcher, deren Lochachse jeweils schräg relativ zur Plattenunterseite verläuft, wobei für jedes einzelne Plattenloch sowohl der Elevationswinkel der Lochachse individuell festlegbar ist wie auch der Azimutwinkel, also der Umlaufwinkel in der Ebene der Plattenunterseite. Dies ist insbesondere für Knochenplatten für den maxillo-facialen Bereich, speziell für den zuvor bereits diskutierten interoralen Zugang günstig, oder für einen Zugang mit Hautschnitt (wegen der nicht freien Wahl des Ortes des Hautschnitts) oder für einen transbuccalen Zugang. Durch die Möglichkeit der individuellen Festlegung des Elevationswinkels und des Azimutwinkels kann die Orientierung der einzelnen Lochachsen der Knochenplatte jeweils so sein, dass ein interoraler Zugang, ein Zugang mit Hautschnitt, oder ein transbuccaler Zugang zu allen Plattenlöchern möglich ist, je nach Anwendungsfall. Insbesondere ist es somit auch möglich, Knochenplatten herzustellen, die auf ganz bestimmte Anwendungen zugeschnitten sind, beispielsweise Knochenplatten für Kieferwinkelfrakturen oder Collumfrakturen. Gleichzeitig kann der Chirurg aufgrund der senkrecht zur Lochachse des jeweiligen Lochs geneigten Fläche auf der Plattenoberseite die jeweilige Solleindrehrichtung für die Schraube gut erkennen.

Ein weiteres vorteilhaftes Ausführungsbeispiel der erfindungsgemässen Knochenplatte weist im Bereich um das jeweilige Plattenloch mit der schräg relativ zur Plattenunterseite verlaufenden Lochachse herum zumindest teilweise eine Überhöhung auf. Die Überhöhung ist in Bezug auf das jeweilige Plattenloch dabei jeweils so angeordnet, dass der Kopf einer in das Plattenloch einschraubbaren Knochenschraube in dem entsprechenden Plattenloch versenkt aufgenommen werden kann. Das soll heissen, dass die Überhöhung so angeordnet ist, dass bei einem für eine bestimmte Operation gewählten typischen Zugang der Chirurg einerseits aufgrund der senkrecht zur Lochachse (und damit zur Solleindrehrichtung) verlaufenden Fläche in der Nähe des Plattenlochs auf der Plattenoberseite die Solleindrehrichtung für die Schraube erkennen kann, und andererseits die Schraube beim Eindrehen auch vollständig im Plattenloch versenkt werden kann. Die schräg zur Plattenunterseite verlaufende Lochachse einerseits und die Überhöhung auf der Plattenoberseite andererseits erlauben es, eine Schraube um einen erheblichen Winkel geneigt relativ zu der Normalen auf die Plattenunterseite einzuschrauben und die Schraube dennoch vollständig im Plattenloch zu versenken. Geht man beispielsweise davon aus, dass die Lochachse gegenüber der Normalen um einen Winkel von 20° geneigt ist und geht man weiter davon aus, dass die Ausgestaltung des Plattenlochs es zulässt, die Schraube um ± 15° relativ zur Solleindrehrichtung (die ja bereits um 20° gegenüber der Normalen geneigt ist) zu neigen und die Schraube dennoch vollständig im Plattenloch versenken und ggf. verblocken zu können (siehe z.B. WO 2004/086990), so ergibt sich ein maximaler Winkel von 20° + 15° = 35° gegenüber der Normalen. Dies eröffnet dem Chirurgen erhebliche Vorteile beim Eindrehen der Schraube.

Bei einer Weiterbildung der erfindungsgemässen Knochenplatte umfasst die Knochenplatte auf der Plattenoberseite mehrere Plattenlöcher mit einer Überhöhung, wobei an jedem Plattenloch mit Überhöhung die Überhöhung individuell ausgebildet und angeordnet ist. Während es grundsätzlich möglich wäre, die Überhöhung, die Lochachsen und die senkrecht zu den Lochachsen verlaufenden Flächen auf der Plattenoberseite alle gleich auszubilden und anzuordnen, erlaubt eine individuelle Ausbildung der Überhöhung für jedes einzelne Plattenloch eine auf den jeweiligen Einsatzort hin optimierte Gestaltung der Knochenplatte.

Ein vorteilhaftes Ausführungsbeispiel der erfindungsgemässen Knochenplatte weist mehrere Ösen auf sowie Stege, durch welche die Ösen miteinander verbunden sind. Im Bereich einer oder mehrerer Ösen weist die Platte jeweils eine Überhöhung auf, jedoch nicht im Bereich der Stege zwischen den Ösen. Somit ist die Platte im Bereich der Stege hinreichend flexibel, um gegebenenfalls noch intraoperativ optimal an den Knochen angebogen werden zu können.

Wie bereits erwähnt, können bei einem vorteilhaften Ausführungsbeispiel der erfindungsgemässen Knochenplatte sämtliche Plattenlöcher so ausgebildet sein, dass beim Fixieren der Platte mit Hilfe von Knochenschrauben sämtliche Knochenschrauben interoral einschraubbar sind. Dadurch kann ein Hautschnitt vermieden werden, selbst dann, wenn beispielsweise mehr oder weniger der ganze Unterkiefer von einer Knochenplatte abgedeckt werden muss.

Wie bereist erwähnt, ist die vorstehend erläuterte erfindungsgemässe Knochenplatte besonders geeignet für Anwendungen im maxillo-facialen Bereich. Die Materialstärke solcher Knochenplatten liegt dabei im Bereich von 0.5 mm bis 2.5 mm, insbesondere im Bereich von 0.5 mm bis 1.6 mm (damit ist die generelle Materialstärke der Knochenplatte gemeint und nicht die Materialstärke an einem überhöhten Bereich). Allerdings ist die Erfindung keineswegs auf solche verhältnismässig "dünnen" Knochenplatten beschränkt, die im maxillo-facialen Bereich zur Anwendung kommen. Vielmehr ist es auch möglich, erfindungsgemässe Knochenplatten mit grösseren Materialstärken (beispielsweise bis hin zu einer Materialstärke von 6 mm) herzustellen, die dann beispielsweise im Bereich der Orthopädie (Gliedmassen, Wirbelsäule, Becken, etc.) zum Einsatz kommen können, wo häufig massivere Knochenplatten erforderlich sind.

Bei einem weiteren Ausführungsbeispiel der erfindungsgemässen Knochenplatte ist die Innenwand des Plattenlochs bzw. der Plattenlöcher mit einer Verblockungskontur versehen, welche mit einer in das Plattenloch einschraubbaren Knochenschraube derart zusammenwirkt, dass diese im Plattenloch verblockbar ist. Dies erlaubt eine winkelstabile Sicherung der Knochenschraube im Plattenloch. Beispiele für eine solche Verblockungskontur sind der in der WO-A-01/19268 gezeigte umlaufende Grat oder auch Verblockungskonturen, wie sie in der WO-A-00/66012 gezeigt sind.

Bei einem speziellen Ausführungsbeispiel der erfindungsgemässen Knochenplatte ist die Verblockungskontur an der Innenwand des Plattenlochs so ausgebildet, dass eine in das Plattenloch einschraubbare Knochenschraube mit einer entsprechend ausgebildeten Klemmfläche am Schraubenkopf durch Verblockung des Schraubenkopfs mit der Verblockungskontur im Plattenloch verblockbar ist. Eine solche Verblockungskontur ist beispielsweise in der WO-A-2004/086990 beschrieben.

Die erfindungsgemässe Knochenplatte ist vorzugsweise aus einem biokompatiblen Material hergestellt, beispielsweise aus Titan oder einer Titanlegierung. Das biokompatible Material kann auch ein bioresorbierbares Material sein, wie z.B. HA/PLLA (hydroxyl apatite/poly(L-lactic acid); PLLA/PGA (poly(L-lactic acid)/polyglycolic acid); PLLA/PDLLA/TMC (poly(L-lactic acid/poly-(D,L-lactic acid /trimethylene carbonate); PLLA/PDLLA (poly(L-lactic acid/Poly-(D,L-lactic acid).

Weitere vorteilhafte Aspekte ergeben sich aus der nachfolgenden Beschreibung von Ausführungsbeispielen der erfindungsgemässen Knochenplatte mit Hilfe der Zeichnung. Es zeigen:
- Fig. 1: ein erstes Ausführungsbeispiel der erfindungsgemässen Knochenplatte in perspektivischer Darstellung
- Fig. 2: die Knochenplatte aus Fig. 1 in Aufsicht,
- Fig. 3: die Knochenplatte aus Fig. 1 in Seitenansicht, mit einer Öse im Schnitt,
- Fig. 4: die Knochenplatte aus Fig. 1 mit eingesetzten Knochenschrauben, in Aufsicht,
- Fig. 5: die Knochenplatte aus Fig. 1 an einem Knochen fixiert, mit zwei Ösen und Schrauben in Schnittdarstellung,
- Fig. 6: die Knochenplatte aus Fig. 1, fixiert an einem Unterkiefer zur Versorgung einer Kieferwinkelfraktur,
- Fig. 7: ein weiteres Ausführungsbeispiel einer erfindungsgemässen Knochenplatte zur Versorgung einer Collumfraktur des *ramus mandibulae* (interoraler Zugang)
- Fig. 8: ein weiteres Ausführungsbeispiel einer erfindungsgemässen Knochenplatte zur Versorgung einer Kieferwinkelfraktur, mit einer Öse zur Befestigung am Alveolarkamm,
- Fig. 9: ein weiteres Ausführungsbeispiel einer erfindungsgemässen Knochenplatte in Form einer Rekonstruktionsplatte zur Versorgung von Mehrfachfrakturen am Unterkiefer,
- Fig. 10: ein weiteres Ausführungsbeispiel einer erfindungsgemässen Knochenplatte mit Lochachsen mit verschiedenen Elevations-winkeln (teilweise in Schnittdarstellung)
- Fig. 11: das Ausführungsbeispiel der Knochenplatte aus Fig. 10 in Aufsicht,
- Fig. 12: ein weiteres Ausführungsbeispiel einer erfindungsgemässen Knochenplatte, welche z.B. für die Versorgung von Frakturen im Bereich des distalen radius (Speiche) geeignet ist,
- Fig. 13: ein weiteres Ausführungsbeispiel einer Knochenplatte, welche z.B. für die Versorgung von Frakturen des *calcaneus* (Fersenbein) geeignet ist
- und:
- Fig. 14: das Ausführungsbeispiel der erfindungsgemässen Knochenplatte aus Fig. 7 (Zugang mit Hautschnitt).

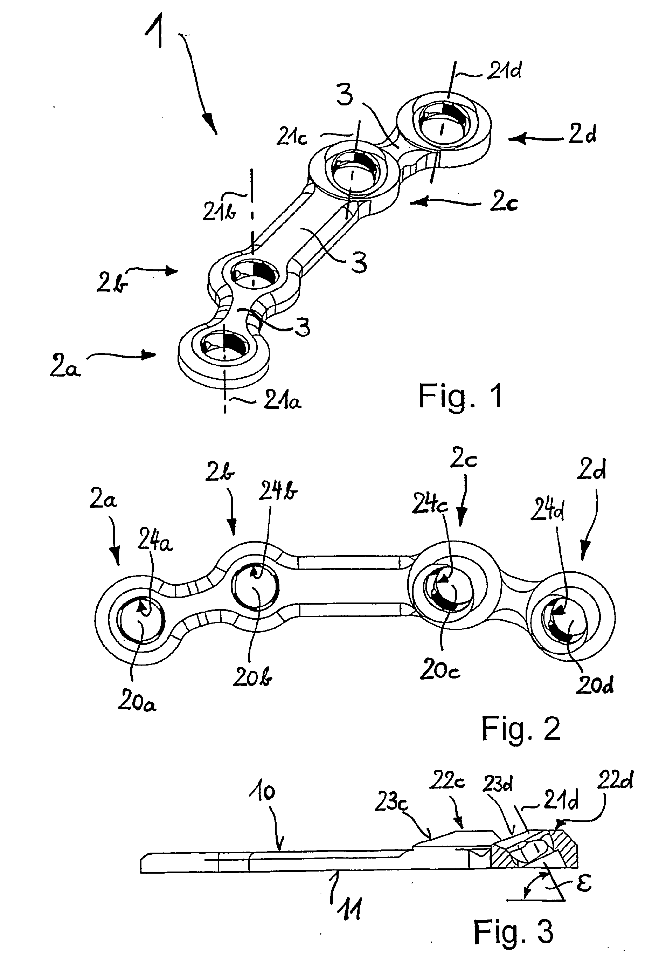

In **Fig. 1, Fig. 2 und Fig. 3** erkennt man ein erstes Ausführungsbeispiel einer erfindungsgemässen Knochenplatte 1. Die Knochenplatte 1 hat eine Plattenoberseite 10 und eine Plattenunterseite 11 (siehe Fig. 3). Ausserdem umfasst sie vier Ösen 2a,2b,2c und 2d, die jeweils durch Stege 3 miteinander verbunden sind. In den Ösen 2a-2d sind Plattenlöcher 20a-20d vorgesehen, durch welche hindurch von der Plattenoberseite 10 her Knochenschrauben in den darunter liegenden Knochen eingeschraubt werden können. Dies wird weiter unten noch genauer erläutert.

Der zwischen den beiden inneren Ösen 2b und 2c gelegene Steg 3 ist massiver ausgebildet als die Stege zwischen den Ösen 2a,2b bzw. 2c,2d. Dieser massivere Steg 3 kommt bei der Fixation der Knochenplatte am Knochen bei der Versorgung einer Fraktur über der Fraktur zu liegen (wird noch erläutert) und stabilisiert die Fraktur, während die Platte im Bereich der beiden äusseren Ösen 2a,2d aufgrund des dünneren Stegs besser an den Knochen angebogen werden kann.

Ebenfalls gut zu erkennen ist (Fig. 1), dass die Lochachsen 21a und 21b der Plattenlöcher 20a und 20b senkrecht zur Plattenunterseite (und auch senkrecht zu der dort parallel verlaufenden Plattenoberseite) verlaufen, während dies für die Lochachsen 21c und 21d der Plattenlöcher 20c und 20d nicht gilt. Da die jeweilige Lochachse immer auch gleichzeitig die "Solleindrehrichtung" für eine Knochenschraube angibt, verläuft die Solleindrehrichtung für die Schrauben in die Plattenlöcher 20c und 20d schräg zur Plattenunterseite 11. Dies wird besonders gut am Beispiel der Lochachse 21d in Fig. 3 ersichtlich, die einen Elevationswinkel ε mit der Ebene der Plattenunterseite 11 einschliesst (die Ebene der Plattenunterseite 11 verläuft in Fig. 3 senkrecht zur Papierebene). Zusätzlich ist es auch möglich, den Azimutwinkel der Lochachse, also den Umlaufwinkel innerhalb der Ebene der Plattenunterseite 11, entsprechend dem geplanten Einsatzzweck einer Knochenplatte zu wählen (also statt wie in Fig. 3 von links oben schräg nach rechts unten könnte die Lochachse 21d auch schräg von rechts oben nach links unten verlaufen, was einem Azimutwinkelunterschied von 180° entspräche). Dies ist grundsätzlich für jedes einzelne Plattenloch möglich, hier allerdings nur bei zwei Plattenlöchern, nämlich bei den Plattenlöchern 20c und 20d, verwirklicht, was abhängig vom jeweiligen Einsatzzweck der Knochenplatte sein kann.

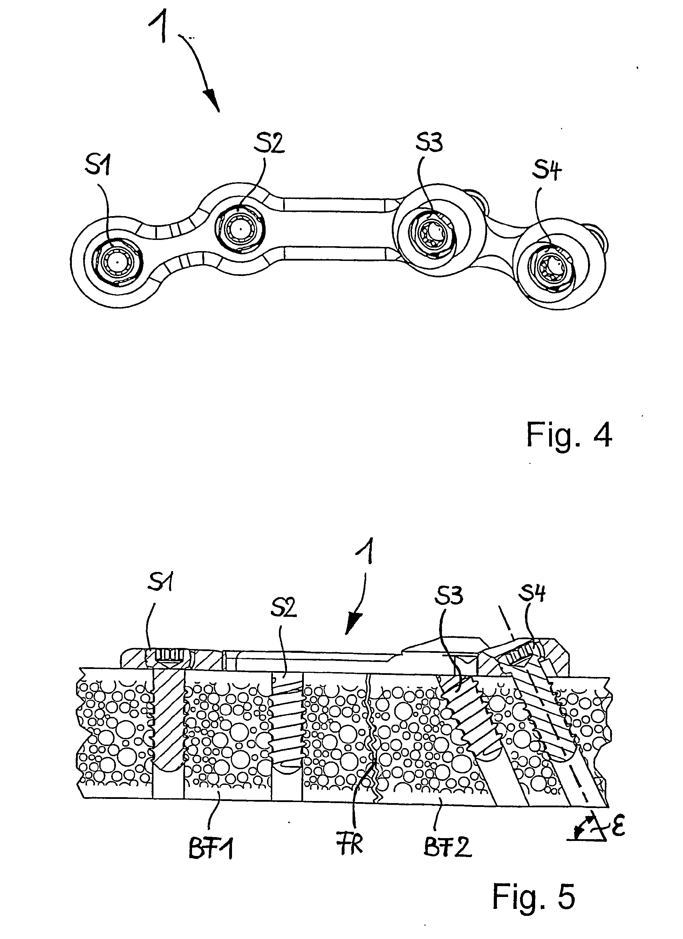

Weiterhin erkennt man, dass im Bereich um die beiden Plattenlöcher 20c bzw. 20d herum, also um die Plattenlöcher mit den schräg verlaufenden Lochachsen 21c bzw. 21d, auf der Plattenoberseite 10 eine Überhöhung 22c bzw. 22d vorgesehen ist. Die Überhöhung kann ebenfalls an jedem Plattenloch individuell ausgebildet und angeordnet sein. Die Überhöhung gewährleistet, dass der Schraubenkopf einer Knochenschraube beim Einschrauben in der Solleindrehrichtung oder nahezu in dieser Richtung trotz der geringen Materialstärke der Knochenplatte, die für Knochenplatten für die Anwendung im maxillo-facialen Bereich typischerweise eine Materialstärke im Bereich von 0.5 mm bis 2.5 mm aufweist, vorzugsweise im Bereich von 0.5 mm bis 1.6 mm, vollständig versenkt aufgenommen werden kann (siehe z.B. Fig. 5, Knochenschraube ganz rechts).

Ausserdem erkennt man, dass im Bereich um das Plattenloch 20c bzw. 20d herum die Plattenoberseite wenigstens teilweise nicht parallel zur Plattenunterseite verläuft. Dies gilt insbesondere für die (Teil-)Fläche 23c bzw. 23d, die senkrecht zur Lochachse 21c bzw. 21d verläuft. Diese Fläche 23c bzw. 23d ist für den Chirurgen bei der Fixation der Knochenplatte nützlich, weil sie dem Chirurgen die Solleindrehrichtung andeutet. Damit ist es für den Chirurgen erheblich einfacher, auch bei einem beengten Operationsfeld, die Solleindrehrichtung zu erkennen und die Schraube entsprechend einzudrehen.

Schliesslich erkennt man noch, dass die Innenwand der einzelnen Plattenlöcher 20a-20d mit einer Verblockungskontur 24a-24d versehen ist (siehe Fig. 2), die es erlaubt, den Schraubenkopf einer speziellen Knochenschraube mit der Verblockungskontur im Plattenloch zu verblocken. Die Verblockungskontur ist in Fig. 3 noch besser zu erkennen - sowohl die Verblockungskontur als auch ein zugehöriger, entsprechend ausgebildeter Schraubenkopf ist aber prinzipiell aus der WO-A-2004/086990 bekannt, deren diesbezügliche Offenbarung hiermit inkorporiert wird. Eine weitere Beschreibung der Verblockungskontur und der zugehörigen Klemmfläche am Schraubenkopf erübrigt sich somit.

**Fig. 4** zeigt die Knochenplatte 1 aus Fig. 1, allerdings mit eingesetzten Knochenschrauben S1,S2,S3 und S4, wobei die Knochenschrauben S1 bis S4 in der Solleindrehrichtung in die Plattenlöcher eingesetzt sind. Man erkennt aus der Aufsicht in Fig. 4, dass die Lochachsen der beiden rechten Plattenlöcher von einer senkrecht auf die Plattenunterseite stehenden Richtung abweicht, denn man erkennt trotz einer Aufsicht auf die Platte die Enden des Schraubenschafts der Knochenschrauben S3 und S4.

**Fig. 5** zeigt nun schematisch die an einem Knochen mit einer Fraktur FR fixierte Knochenplatte 1, oder besser gesagt, die an zwei Knochenfragmenten BF1 und BF2 fixierte Knochenplatte 1, wobei jeweils eines der beiden Typen von Plattenlöchern - ein Plattenloch ohne Überhöhung und ein Plattenloch mit Überhöhung, im Schnitt dargestellt sind, und zwar bei in den Knochen eingeschraubter Knochenschraube S1 bzw. S4. Man kann erkennen, dass trotz Einschrauben der Knochenschraube S4 unter dem Elevationswinkel e (der Azimutwinkel sei hier ausser Acht gelassen) der Schraubenkopf vollständig versenkt ist.

Die in **Fig. 4** und Fig. 5 dargestellte vorteilhafte Eingriffskontur im Schraubenkopf für ein Eindrehwerkzeug sowie ein entsprechendes Eindrehwerkzeug (Schraubendreher) sind beispielsweise bekannt aus der DE 10 2004 026 769 A1.

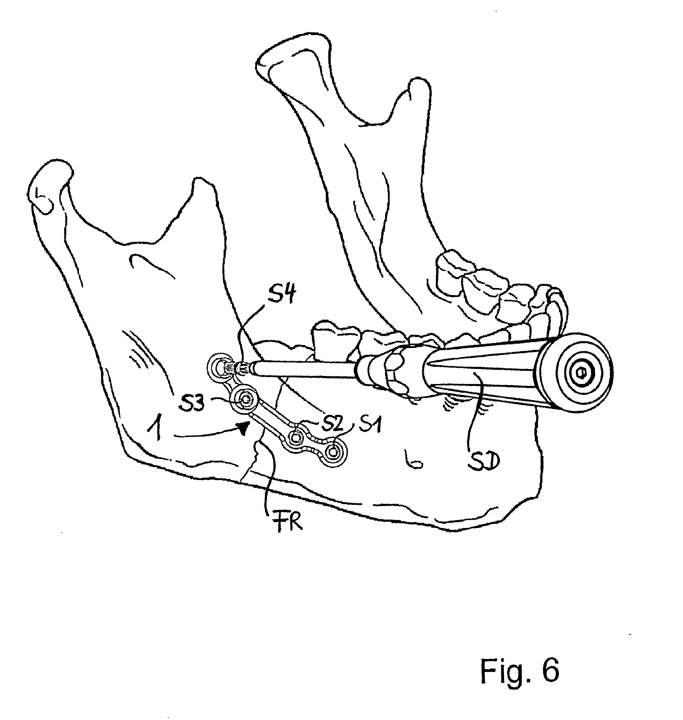

In **Fig. 6** erkennt man die Knochenplatte 1 in der Anwendung, nämlich bei der Versorgung einer Kieferwinkelfraktur des Unterkiefers (*mandibula)*. Während die beiden Knochenschrauben S1 und S2 ohne weiteres interoral in die gut zugänglichen Plattenlöcher eingeschraubt sind, kann man nun den besonderen Vorteil der erfindungsgemässen Knochenplatte hier erkennen. Die Lochachsen der beiden Plattenlöcher, die im Bereich des aufsteigenden *ramus mandibulae* zu liegen kommen, sind hier so angeordnet, dass sie ebenfalls bei einem interoralen Zugang (also ohne Hautschnitt) für den Chirurgen gut zugänglich sind. Dazu sind die Überhöhungen und die Schrägflächen (siehe Erläuterungen zu Fig. 1-3) der beiden Plattenlöcher für die Knochenschrauben S3 und S4 auf spezielle Art und Weise angeordnet, nämlich so, dass einerseits der Chirurg erkennen kann, welches die Solleindrehrichtung ist und andererseits ein rein interoraler Zugang möglich ist. Dies ist durch die räumliche Anordnung des zu diesem Zweck symbolisch dargestellten Schraubendrehers SD beim Eindrehen der Schraube S4 ersichtlich.

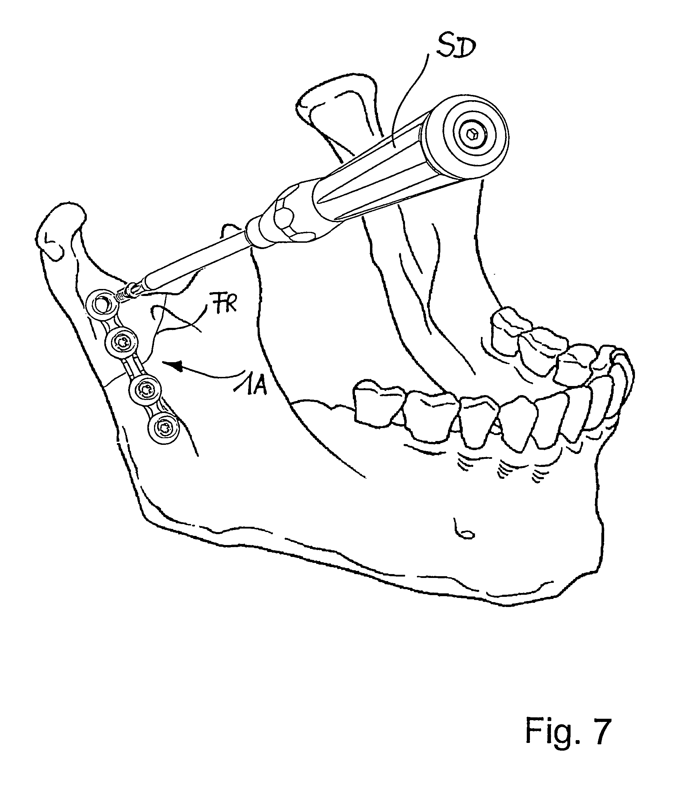

In **Fig. 7** erkennt man ein zweites Ausführungsbeispiel der erfindungsgemässen Knochenplatte 1A. Die Knochenplatte 1A unterscheidet sich im wesentlichen von der Knochenplatte 1 dadurch, dass sie geradlinig ausgebildet ist, und dass jedes einzelne Plattenloch mit einer Überhöhung versehen ist. Das hat seinen Grund darin, dass die Knochenplatte 1A - wie in Fig. 6 gezeigt - besonders für Frakturen FR im subkondylären Bereich des aufsteigenden *ramus mandibulae* (bzw. Collumfrakturen) geeignet ist. Dort ist aber ein interoraler Zugang ohne Hautschnitt nur beschränkt möglich, und auch nur dann, wenn die Überhöhungen und die Lochachsen der Plattenlöcher so orientiert sind, dass die Schrauben S1 bis S4 auch tatsächlich bei einem rein interoralen Zugang in der Sollrichtung einschraubbar und versenkbar sind und die Schrägfläche dem Chirurgen die Solleindrehrichtung andeuten. Dies soll erneut durch die räumliche Anordnung des Schraubendrehers SD ersichtlich werden.

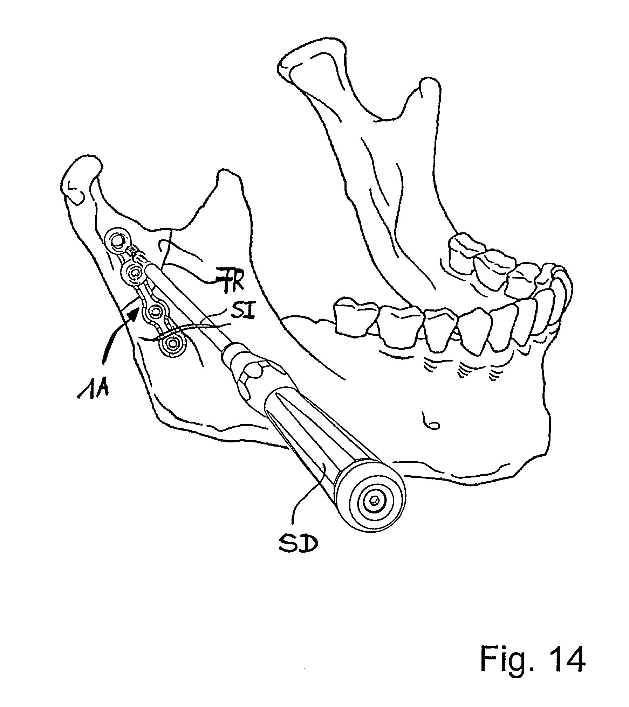

In Fällen, in denen kein interoraler Zugang möglich ist oder aus anderen Gründen nicht erfolgen kann oder soll, kann beispielsweise ein Zugang mit Hautschnitt erfolgen. Aber auch bei einem Zugang mit Hautschnitt kann der Ort des Hautschnitts aufgrund des Verlaufs der Nerven im maxillo-facialen Bereich nicht einfach so gewählt werden, dass man ohne weiteres den Schraubendreher senkrecht durch den Hautschnitt hindurch führen kann und dann auf die Knochenplatte 1 stösst. Dies gilt ganz besonders für Collumfrakturen, wie Sie bereits in **Fig. 7** gezeigt sind (dort aber bei rein interoralem Zugang ohne Hautschnitt), aber auch bei einem Zugang mit Hautschnitt SI, wie dies in **Fig. 14** gezeigt ist, wo sowohl der Hautschnitt SI dargestellt ist als auch der Schraubendreher SD, sodass man aus **Fig. 14** erkennen kann, dass der Schraubendreher SD nur unter einem Winkel abweichend von der Normalen zu den Plattenlöchern der Knochenplatte 1 gelangen kann, um die Schrauben einzudrehen. Dies gilt in entsprechender Weise auch für einen transbuccalen Zugang (siehe weiter oben).

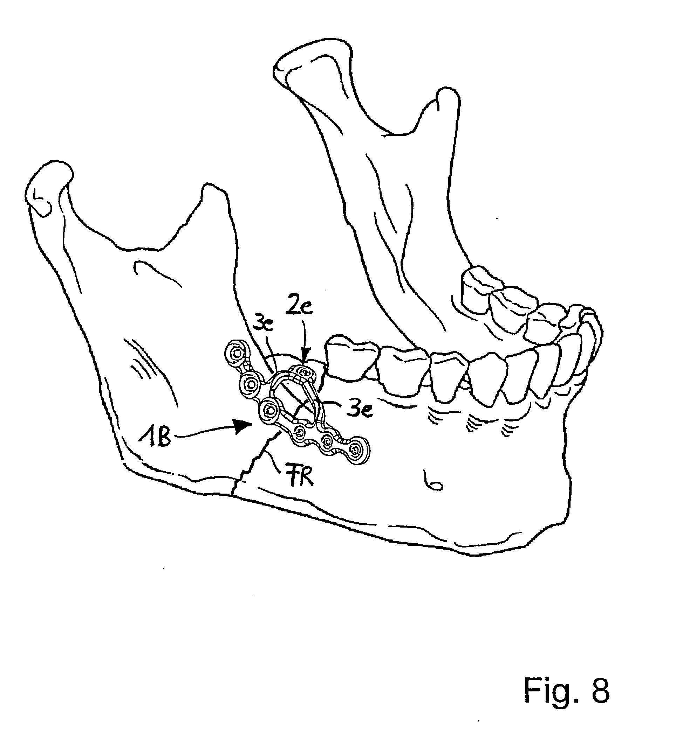

In **Fig. 8** erkennt man ein drittes Ausführungsbeispiel der erfindungsgemässen Knochenplatte 1B, welches grundsätzlich dem ersten Ausführungsbeispiel der Knochenplatte 1 sehr ähnlich ist, allerdings jeweils drei Plattenlöcher ohne Überhöhung und drei Plattenlöcher mit Überhöhung für diesseits bzw. jenseits einer Kieferwinkelfraktur FR aufweist. Zusätzlich ist jedoch noch eine Öse 2e vorgesehen, die am hinteren Ende des Alveolarkamms fixiert werden kann, sowie zwei zusätzliche Stege 3e zur Verbindung dieser Öse 2e mit dem restlichen Korpus der Knochenplatte 1B. Bezüglich der Orientierung der Lochachsen, die Anordnung und Ausbildung der Überhöhungen und der Solleindrehrichtung bzw. des möglichen rein interoralen Zugangs gelten die Ausführungen weiter oben in gleicher Weise.

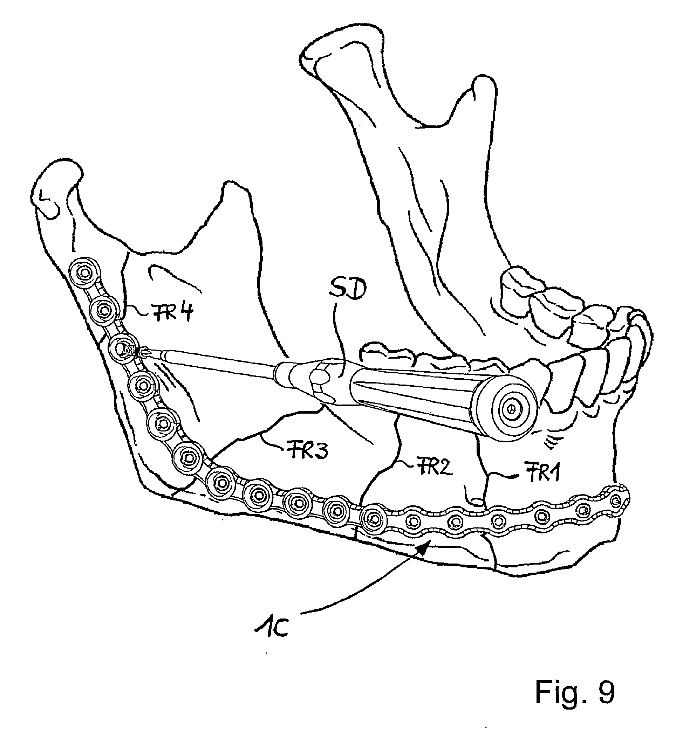

In **Fig. 9** erkennt man ein viertes Ausführungsbeispiel der erfindungsgemässen Knochenplatte 1C in Form einer Rekonstruktionsplatte zur gleichzeitigen Versorgung von Mehrfachfrakturen FR1,FR2,FR3,FR4, zum Zwecke der Rekonstruktion des Unterkiefers. Bei diesem Ausführungsbeispiel erkennt man eine Vielzahl von Ösen und die Ösen verbindende Stege, damit sich die Platte möglichst gut zu der gewünschten Form biegen lässt. Im Frontbereich sind die Plattenlöcher ohne Überhöhungen ausgeführt, weil dieser interoral gut zugänglich ist, während sie im seitlichen und hinteren Bereich der Knochenplatte, wo der interorale Zugang schwieriger ist, in unterschiedlichen Winkeln überhöht ausgebildet sind. In Bezug auf die Orientierung der Lochachsen, die Anordnung und Ausbildung der Überhöhungen und die Solleindrehrichtung bzw. des möglichen rein interoralen Zugangs gelten die Ausführungen von weiter oben in gleicher Weise. Zur Verdeutlichung ist in Fig. 9 erneut eine Schraubendreher SD dargestellt.

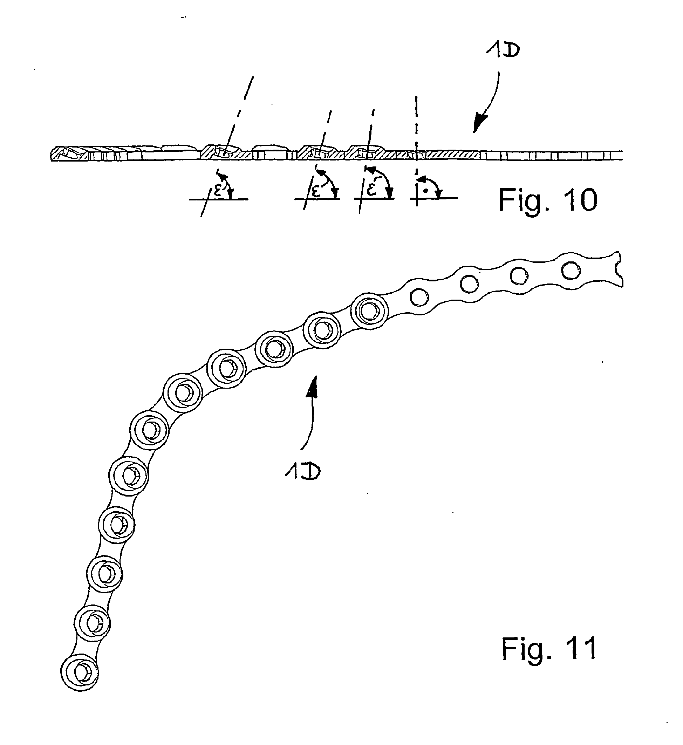

In **Fig. 10** und **Fig. 11** ist ein weiteres Ausführungsbeispiel einer erfindungsgemässen Knochenplatte 1D dargestellt. Bei diesem Ausführungsbeispiel ist insbesondere zu erkennen, dass der Elevationswinkel ε der Lochachsen an den verschiedenen Plattenlöchern unterschiedlich sein kann und prinzipiell für jedes einzelne Loch separat festgelegt werden kann.

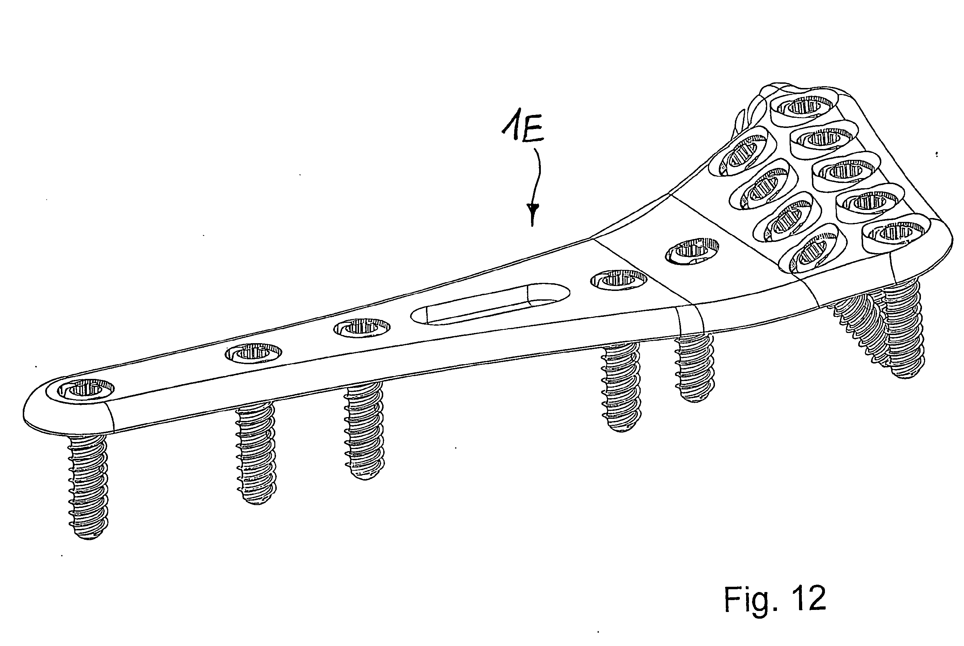

**Fig. 12** zeigt ein weiteres Ausführungsbeispiel einer erfindungsgemässen Knochenplatte 1E, welche z.B. für die Versorgung von Frakturen im Bereich des distalen *radius* (Speiche) geeignet ist. Auch hier erkennt man die teilweise überhöhten Plattenlöcher der Knochenplatte in demjenigen Bereich, der zur Befestigung nahe zum Handgelenk am distalen Ende der Speiche vorgesehen ist. Auch hier gelten sinngemäss die gleichen Betrachtungen im Hinblick auf die Variabilität der Ausgestaltung der Knochenlatte, wie sie schon anhand der weiter oben detailliert erläuterten Ausführungsbeispiele der erfindungsgemässen Knochenplatte schon angestellt sind.

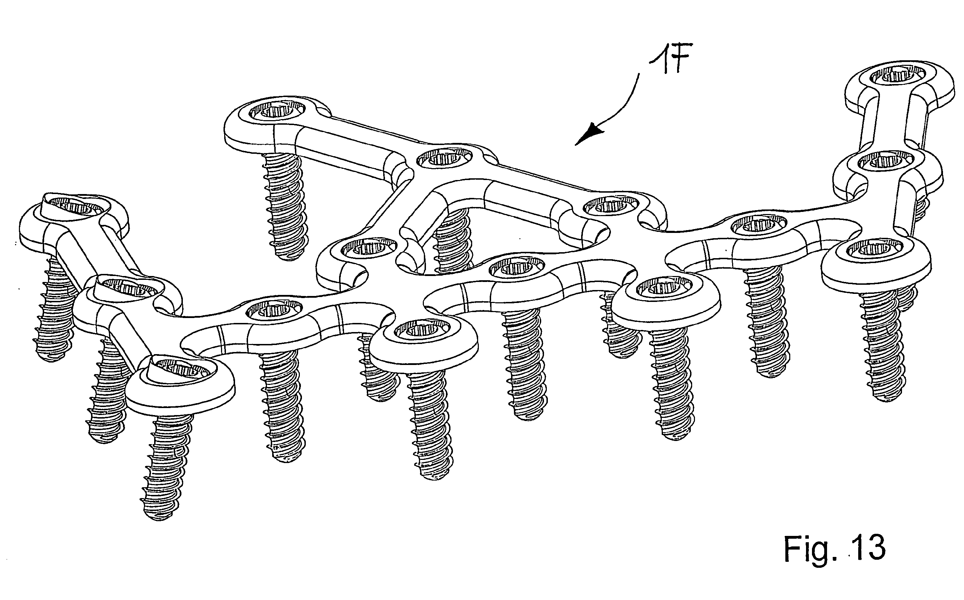

Schliesslich ist in **Fig. 13** ein weiteres Ausführungsbeispiel einer Knochenplatte 1F dargestellt, welche z.B. für die Versorgung von Frakturen des *calcaneus* (Fersenbein) geeignet ist. Auch bezüglich dieses Ausführungsbeispiels der Knochenplatte gelten sinngemäss die gleichen Betrachtungen im Hinblick auf die Variabilität der Ausgestaltung der Knochenplatte.

Zu den gezeigten Ausführungsbeispielen sind grundsätzlich beliebig viele Variationen denkbar, alleine deshalb, weil durch die Möglichkeit der individuellen Anordnung und Ausbildung der Lochachsen, Überhöhungen und Schrägflächen sich die Knochenplatten jeweils optimal auf ihren Einsatzzweck hin zuschneiden lässt.

Die erfindungsgemässe Knochenplatte ist typischerweise aus einem biokompatiblen Material, wie beispielsweise Titan oder Titanlegierungen, hergestellt. Bioresorbierbare Materialien, wie beispielsweise HA/PLLA (hydroxyl apatite/poly(L-lactic acid); PLLA/PGA (poly(L-lactic acid)/polyglycolic acid); PLLA/PDLLA/TMC (poly(L-lactic acid/poly-(D,L-lactic acid /trimethylene carbonate); PLLA/PDLLA (poly(L-lactic acid/Poly-(D,L-lactic acid) können aber ebenfalls in Betracht kommen, vor allen Dingen dann, wenn dies von den Anforderungen an die Knochenplatte möglich ist und dann später auf eine Entfernung der Knochenplatte und die damit verbundenen Beeinträchtigungen für den Patienten verzichtet werden kann.

## Patentansprüche

1. Knochenplatte (1;1A;1B;1C;1D;1E;1F) mit mindestens einem Plattenloch (20a,20b,20c,20d), dessen Lochachse (21a,21b,21c,21d) schräg relativ zur Plattenunterseite (11) verläuft, also einen Elevationswinkel (ε) relativ zur Ebene der Plattenunterseite (11) aufweist, der von 90° verschieden ist, und bei welcher Platte die Plattenoberseite (10) zumindest im Bereich um das Plattenloch (20c,20d) herum wenigstens teilweise nicht parallel zur Plattenunterseite (11) verläuft und das Plattenloch so ausgebildet ist, dass eine in das Plattenloch (20c,20d) eingeschraubte Knochenschraube (S3,S4) unter einem Winkel aus einem vordefinierten Winkelbereich um den Elevationswinkel (ε) der Lochachse (21c,21d) des Plattenlochs (20c,20d) herum in dem Plattenloch (20c,20d) verblockbar ist, und zwar nur durch Zusammenwirken des speziell ausgebildeten Plattenlochs mit der Knochenschraube, also ohne zusätzliche weitere Hilfsmittel, **dadurch gekennzeichnet, dass** die Materialstärke der Knochenplatte im Bereich von 0.5 mm bis 2.5 mm liegt, insbesondere im Bereich von 0.5 mm bis 1.6 mm, und dass die Plattenoberseite zumindest im Bereich um das Plattenloch (20c,20d) herum senkrecht zur Lochachse (21d,21d) verläuft, wobei hierzu auf der Plattenoberseite (10) im Bereich um das jeweilige Plattenloch (20c,20d) mit der schräg relativ zur Plattenunterseite verlaufenden Lochachse (21c,21d) herum zumindest teilweise eine Überhöhung (22c,22d) vorgesehen ist, die in Bezug auf das jeweilige Plattenloch (20c,20d) so angeordnet ist, dass der Kopf einer in das Plattenloch (20c,20d) einschraubbaren Knochehschraube (S3,S4) in dem entsprechenden Plattenloch (20c,20d) versenkt aufgenommen werden kann.

2. Knochenplatte nach Anspruch 1, bei welcher der vordefinierte Winkelbereich um den Elevationswinkel der Knochenplatte herum etwa 15° beträgt.

3. Knochenplatte nach einem der Ansprüche 1 oder 2, welche mehrere Plattenlöcher (20c,20d) umfasst, deren Lochachse (21c,21d) jeweils schräg relativ zur Plattenunterseite (11) verläuft, wobei für jedes einzelne Plattenloch (20c,20d) sowohl der Elevationswinkel (ε) der Lochachse individuell festlegbar ist wie auch der Azimutwinkel, also der Umlaufwinkel in der Ebene der Plattenunterseite (11).

4. Knochenplatte nach einem der vorangehenden Ansprüche, welche mehrere Plattenlöcher (20c,20d) mit einer Überhöhung (22c,22d) umfasst, wobei an jedem Plattenloch mit Überhöhung die Überhöhung individuell ausgebildet und angeordnet ist.

5. Knochenplatte nach einem der vorangehenden Ansprüche, welche mehrere Ösen (2a,2b,2c,2d;2e) aufweist sowie Stege (3;3e), durch welche die Ösen miteinander verbunden sind, und dass die Platte im Bereich einer oder mehrerer Ösen jeweils eine Überhöhung (20c,20d) aufweist, jedoch nicht im Bereich der Stege (3;3e) zwischen den Ösen (2a,2b,2c,2d;2e).

6. Knochenplatte nach einem der vorangehenden Ansprüche, bei welcher sämtliche Plattenlöcher (20a,20b,20c,20d) so ausgebildet sind, dass beim Fixieren der Platte mit Hilfe von Knochenschrauben (S1,S2,S3,S4) sämtliche Knochenschrauben (S1,S2,S3,S4) interoral einschraubbar sind.

7. Knochenplatte nach einem der vorangehenden Ansprüche, bei welcher die Innenwand des Plattenlochs bzw. der Plattenlöcher (20a,20b,20c,20d) mit einer Verblockungskontur (24a,24b,24c,24d) versehen ist, welche mit der in das jeweilige Plattenloch (20a,20b,20c,20d) einschraubbaren Knochenschraube (S1,S2,S3,S4) derart zusammenwirkt, dass diese im Plattenloch verblockbar ist.

8. Knochenplatte nach Anspruch 7, bei welcher die Verblockungskontur (24a,24b,24c,24d), an der Innenwand des Plattenlochs (20a,20b,20c.20d) so ausgebildet ist, dass eine in das Plattenloch (20a,20b,20c,20d) einschraubbare Knochenschraube (S1,S2,S3,S4) mit einer entsprechend ausgebildeten Klemmfläche am Schraubenkopf durch Verblockung des Schraubenkopfs mit der Verblockungskontur (24a,24b,24c,24d) im Plattenloch (20a,20b,20c,20d) verblockbar ist.

9. Knochenplatte nach einem der vorangehenden Ansprüche, welche aus einem biokompatiblen Material hergestellt ist.

10. Knochenplatte nach Anspruch 9, bei welcher das biokompatible Material eine bioresorbierbares Material ist.

## Claims

1. Bone plate (1; 1A; 1B; 1C; 1D; 1E; 1F) with at least one plate hole (20a, 20b, 20c, 20d) whose hole axis (21a, 21b, 21c, 21d) runs obliquely relative to the underside (11) of the plate, that is to say has an elevation angle (ε), relative to the plane of the underside (11) of the plate, which is different from 90°, and in which plate the upper side (10) of the plate runs at least partially non-parallel to the underside (11) of the plate, at least in the area around the plate hole (20c, 20d), and the plate hole is designed such that a bone screw (S3, S4), which is screwed into the plate hole (20c, 20d), can be blocked in the plate hole (20c, 20d) at an angle chosen from a predefined angle range around the elevation angle (ε) of the hole axis (21c, 21d) of the plate hole (20c, 20d), specifically only by cooperation of the specially designed plate hole with the bone screw without additional other auxiliary means, **characterized in that** the material thickness of the bone plate is in the range of 0.5 mm to 2.5 mm, in particular in the range of 0.5 mm to 1.6 mm, and **in that** the upper side of the plate, at least in the area around the plate hole (20c, 20d), runs perpendicular to the hole axis (21c, 21d), for which purpose an elevation (22c, 22d) is provided at least partly on the top side (10) of the plate in the area around the respective plate hole (20c, 20d) with the hole axis (21c, 21d) running obliquely in relation to the underside of the plate, which elevation (22c, 22d) is arranged in relation to the respective plate hole (20c, 20d) such that the head of a bone screw (S3, S4), which can be screwed into the plate hole (20c, 20d), can be received and countersunk in the corresponding plate hole (20c, 20d).

2. Bone plate according to Claim 1, in which the predefined angle range around the elevation angle of the bone plate is approximately 15°.

3. Bone plate according to one of Claims 1 and 2, comprising a plurality of plate holes (20c, 20d) whose hole axis (21c, 21d) in each case runs obliquely relative to the underside (11) of the plate, wherein for each individual plate hole (20c, 20d), both the elevation angle (e) of the hole axis and also the azimuth angle, that is to say the rotation angle in the plane of the underside (11) of the plate can be individually determined.

4. Bone plate according to one of the preceding claims, comprising a plurality of plate holes (20c, 20d) with an elevation (22c, 22d), wherein the elevation is designed and arranged individually on each plate hole with an elevation.

5. Bone plate according to one of the preceding claims, comprising a plurality of eyes (2a, 2b, 2c, 2d; 2e) and webs (3; 3e) through which the eyes are connected to one another, and the plate has an elevation (20c, 20d) in the area of one or more eyes, but not in the area of the webs (3; 3e) between the eyes (2a, 2b, 2c, 2d; 2e).

6. Bone plate according to one of the preceding claims, in which all the plate holes (20a, 20b, 20c, 20d) are designed such that, when fixing the plate with the aid of bone screws (S1, S2, S3, S4), all the bone screws (S1, S2, S3, S4) can be screwed in intraorally.

7. Bone plate according to one of the preceding claims, in which the inside wall of the plate hole or plate holes (20a, 20b, 20c, 20d) is provided with a blocking contour (24a, 24b, 24c, 24d) which cooperates with the bone screw (S1, S2, S3, S4), which can be screwed into the respective plate hole (20a, 20b, 20c, 20d), in such a way that the bone screw (S1, S2, S3, S4) can be blocked in the plate hole.

8. Bone plate according to Claim 7, in which the blocking contour (24a, 24b, 24c, 24d) is designed on the inside wall of the plate hole (20a, 20b, 20c, 20d) such that a bone screw (S1, S2, S3, S4), which can be screwed into the plate hole (20a, 20b, 20c, 20d) and which has a suitably designed clamping surface on the screw head, can be blocked in the plate hole (20a, 20b, 20c, 20d) by blocking the screw head with the blocking contour (24a, 24b, 24c, 24d).

9. Bone plate according to one of the preceding claims, wherein the bone plate is made of a biocompatible material.

10. Bone plate according to Claim 9, wherein the biocompatible material is a bioresorbable material.

## Revendications

1. Plaque d'ostéosynthèse (1; 1A; 1B; 1C; 1D; 1E; 1F) qui comprend au moins un trou de plaque (20a, 20b, 20c, 20d) dont l'axe (21a, 21b, 21c, 21d) s'étend obliquement par rapport à la face inférieure (11) de la plaque et présente donc par rapport au plan de la face inférieure (11) de la plaque un angle d'élévation (ε) différent de 90°,
la face supérieure (10) de cette plaque s'étendant en partie non parallèlement à la face inférieure (11) de la plaque au moins dans la partie qui entoure le trou (20c, 20d) de la plaque, le trou de plaque étant configuré de telle sorte qu'une vis d'ostéosynthèse (S3, S4) vissée dans le trou (20c, 20d) de la plaque peut être bloqué dans le trou (20c, 20d) de la plaque sous un angle d'une plage angulaire prédéfinie autour de l'angle d'élévation (ε) de l'axe (21c, 21d) du trou (20c, 20d) de la plaque, et ce uniquement par coopération du trou de plaque de configuration spéciale avec la vis d'ostéosynthèse et donc sans autre accessoire supplémentaire,
**caractérisée en ce que**
l'épaisseur de la plaque d'ostéosynthèse est comprise dans la plage de 0,5 mm à 2,5 mm et en particulier dans la plage de 0,5 mm à 1,6 mm, et **en ce qu'**au moins dans la partie qui entoure le trou (20c, 20d) de la plaque, la face supérieure de la plaque s'étend perpendiculairement à l'axe (21c, 21d) du trou, et
**en ce que** dans ce but, sur la face supérieure (10) de la plaque, dans la partie qui entoure le trou (20c, 20d) concerné de la plaque et dont l'axe (21c, 21d) s'étend obliquement par rapport à la face inférieure de la plaque, une surélévation (22c, 22d) est disposée sur au moins une partie de la périphérie du trou (20c, 20d) concerné de la plaque, de telle sorte que la tête d'une vis d'ostéosynthèse (S3, S4) qui peut être vissée dans le trou (20c, 20d) de la plaque peut être enfoncée dans le trou correspondant (20c, 20d) de la plaque.

2. Plaque d'ostéosynthèse selon la revendication 1, dans laquelle la plage angulaire prédéfinie autour de l'angle d'élévation de la plaque d'ostéosynthèse est d'environ 15°.

3. Plaque d'ostéosynthèse selon l'une des revendications 1 ou 2, qui présente plusieurs trous de plaque (20c, 20d) dont l'axe (21c, 21d) s'étend chaque fois obliquement par rapport à la face inférieure (11) de la plaque, et dans laquelle tant l'angle d'élévation (ε) de l'axe du trou que l'angle d'azimut, c'est-à-dire l'angle périphérique dans le plan de la face inférieure (11) de la plaque peuvent être définis séparément pour chaque trou (20c, 20d) de la plaque.

4. Plaque d'ostéosynthèse selon l'une des revendications précédentes, qui comprend plusieurs trous de plaque (20c, 20d) dotés d'une surélévation (22c, 22d), la surélévation de chaque trou de plaque doté d'une surélévation étant configurée et disposée individuellement.

5. Plaque d'ostéosynthèse selon l'une des revendications précédentes, qui présente plusieurs oeillets (2a, 2b, 2c, 2d; 2e) ainsi que des nervures (3; 3e) qui relient entre eux les oeillets, tandis que dans la zone occupée par un ou plusieurs oeillets, la plaque présente chaque fois une surélévation (20c, 20d), mais pas dans la zone occupée par les nervures (3; 3e) qui relient les oeillets (2a, 2b, 2c, 2d; 2e).

6. Plaque d'ostéosynthèse selon l'une des revendications précédentes, dans laquelle tous les trous (20a, 20b, 20c, 20d) de la plaque sont configurés de telle sorte que lorsque la plaque est fixée à l'aide de vis d'ostéosynthèse (S1, S2, S3, S4), toutes les vis d'ostéosynthèse (S1, S2, S3, S4) peuvent être vissées en position interorale.

7. Plaque d'ostéosynthèse selon l'une des revendications précédentes, dans laquelle la paroi intérieure du ou des trous (20a, 20b, 20c, 20d) de la plaque est dotée d'un contour de blocage (24a, 24b, 24c, 24d) qui coopère avec la vis d'ostéosynthèse (S1, S2, S3, S4) qui peut être vissée dans chaque trou (20a, 20b, 20c, 20d) de la plaque pour la bloquer dans le trou de la plaque.

8. Plaque d'ostéosynthèse selon la revendication 7, dans laquelle le contour de blocage (24a, 24b, 24c, 24d) formé sur la paroi intérieure du trou (20a, 20b, 20c, 20d) de la plaque est configuré de telle sorte qu'une vis d'ostéosynthèse (S1, S2, S3, S4) dont la tête est dotée d'une surface de serrage de configuration complémentaire et apte à être vissée dans le trou (20a, 20b, 20c, 20d) de la plaque peut être bloquée par blocage de la tête de la vis sur le contour de blocage (24a, 24b, 24c, 24d) prévu dans le trou (20a, 20b, 20c, 20d) de la plaque.

9. Plaque d'ostéosynthèse selon l'une des revendications précédentes, fabriquée en un matériau biocompatible.

10. Plaque d'ostéosynthèse selon la revendication 9, dans laquelle le matériau biocompatible est un matériau biorésorbable.
